(19) Europäisches Patentamt
European Patent Office
Office européen des brevets

(11) EP 2 473 235 B1

(12) **EUROPEAN PATENT SPECIFICATION**

(45) Date of publication and mention of the grant of the patent:
**27.06.2018 Bulletin 2018/26**

(21) Application number: **10771360.4**

(22) Date of filing: **02.09.2010**

(51) Int Cl.:
***A61Q 5/10*** *(2006.01)* ***A61K 8/34*** *(2006.01)*
***A61K 8/49*** *(2006.01)* ***A61Q 5/06*** *(2006.01)*

(86) International application number:
**PCT/EP2010/062909**

(87) International publication number:
**WO 2011/026918 (10.03.2011 Gazette 2011/10)**

(54) **COMPOSITION COMPRISING AN ACID DYE OF INDIGOID TYPE AND DYEING METHOD**

ZUSAMMENSETZUNG MIT EINEM SAUREN INDIGO-FÄRBEMITTEL UND FÄRBEVERFAHREN DAFÜR

COMPOSITION COMPRENANT UN COLORANT ACIDE DU TYPE INDIGOÏDE ET PROCÉDÉ DE COLORATION

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **02.09.2009 FR 0955970**
**14.09.2009 US 242124 P**

(43) Date of publication of application:
**11.07.2012 Bulletin 2012/28**

(73) Proprietor: **L'Oréal**
**75008 Paris (FR)**

(72) Inventors:
- **LALLEMAN, Boris**
**F-75004 Paris (FR)**
- **LAGRANGE, Alain**
**F-77700 Coupvray (FR)**

(74) Representative: **Duvert, Sandra**
**L'Oréal**
**Service DIPI**
**9 Rue Pierre Dreyfus**
**92110 Clichy (FR)**

(56) References cited:
**EP-A2- 0 806 198** **US-A1- 2009 016 975**
**US-A1- 2009 156 562**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 473 235 B1

## Description

The present invention relates to a composition for dyeing human keratinous fibres comprising at least one acid direct dye of indigoid type and at least one specific organic compound and to a dyeing method.

Two main methods are known for dyeing human keratinous fibres and in particular the hair.

The first, known as oxidation dyeing or permanent dyeing, consists in employing one or more oxidation dye precursors, more particularly one or more oxidation bases, optionally in combination with one or more couplers.

Usually, oxidation bases are chosen from ortho- or para-phenylehediamines, ortho- or para-aminophenols, and heterocyclic compounds. These oxidation bases are colourless or weakly coloured compounds which, in combination with oxidizing products, make it possible to arrive, by an oxidative coupling process, at coloured entities which remain trapped inside the fibre.

The shades obtained with these oxidation bases are very often varied by combining them with one or more couplers, the latter being chosen in particular from aromatic meta-diamines, meta-aminophenols, meta-diphenols and certain heterocyclic compounds, such as indole compounds.

The variety of the molecules employed as oxidation bases and couplers makes it possible to obtain a rich palette of colours.

Dyeing of this type also makes it possible to obtain permanent colourings but the use of oxidizing agents can cause damage to keratinous fibres.

The second dyeing method, known as direct or semipermanent dyeing, comprises the application of direct dyes, which are molecules having affinity for the fibres and which colour even in the absence of oxidizing agent added to the compositions comprising them. Given the nature of the molecules employed, the latter remain rather at the surface of the fibre and penetrate to a relatively slight extent into the fibre, compared with the small molecules of oxidation dye precursors.

The direct dyes generally employed are chosen from nitrobenzene, anthraquinone, nitropyridine, azo, methine, azomethine, xanthene, acridine, azine or triarylmethane direct dyes. The chemical entities employed can be nonionic, anionic (acid dyes) or cationic (basic dyes). Direct dyes can also be natural dyes.

The majority of the direct dyes employed have a satisfactory solubility in an aqueous medium and there now exists many dyeing vehicles suitable for employing them.

These compositions comprising one or more direct dyes are applied to the keratinous fibres for a time necessary to obtain the desired colouring and are then rinsed out.

However, the colourings which result therefrom are colourings which are particularly chromatic but temporary or semipermanent due to the nature of the interactions which bind the direct dyes to the keratinous fibre, and their desorption from the surface and/or from the core of the fibre are responsible for their low dyeing power and for their poor resistance to washing operations or to light.

The aim of the present invention is to provide novel compositions for dyeing human hair which respect the nature of the hair and make possible powerful and resistant colourings, in particular for the blues, and which can also give varied shades.

Thus, a subject-matter of the present invention is a dyeing composition comprising:

- one or more acid direct dyes

chosen from the following compounds and their mixtures:

| | |
|---|---|
| 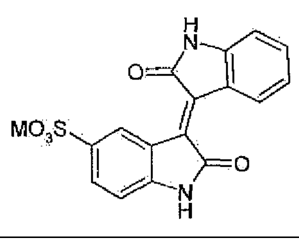 (1) | 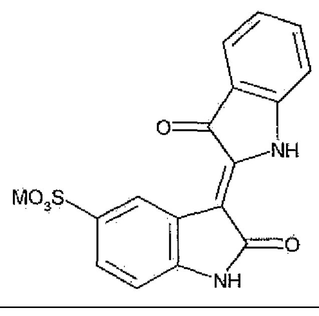 (2) |
| 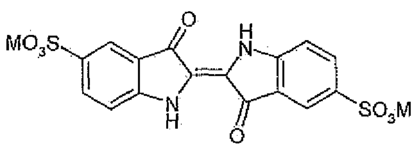 (3) | 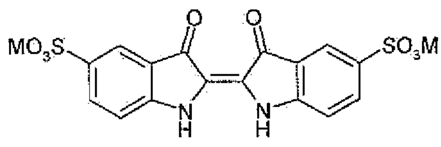 (4) |

(continued)

| | |
|---|---|
| 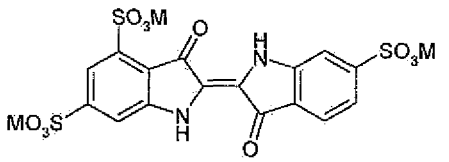 **(5)** | 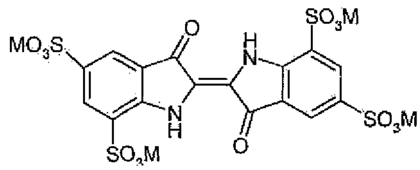 **(6)** |

M representing a hydrogen atom, a sodium ion or a potassium, ion, the content of this (these) acid direct dye(s) being at least 0.01% by weight, with respect to the weight of the composition; and

- one or more organic compounds (II) exhibiting a value for the Hansen solubility parameter δH of between 5 and 16 $MPa^{1/2}$ and with a molecular weight of less than 250 g/mol, at 25°C, said compound being chosen from benzyl alcohol.

More preferably, the dyes correspond to the sodium salts of the compounds (1), (2), (3) and (4) and to the potassium salts of the compounds of formulae (3), (5) and (6) .

According to a particularly advantageous embodiment of the invention, the dye present in the composition of the inventionis the compound (3) more particularly in the sodium salt form (indigo carmine).

The acid direct dye or dyes of formula (1) to (6) generally represent from 0.01 to 20% by weight, preferably from 0.05 to 10% by weight, better still from 0.1 to 5% by weight, of the total weight of the cosmetic composition.

As explained above, the composition according to the invention also comprises one or more specific compounds (II) chosen from benzyl alcohol.

As explained above, this compound (II) exhibit a value for the Hansen solubility parameter δH of between 5 and 16 $MPa^{1/2}$, advantageously between 5 and less than 16 $MPa^{1/2}$, and with a molecular weight of less than 250 g/mol. Preferably, the value for the Hansen solubility parameter δH is between 5 and 15.8 $MPa^{1/2}$, more particularly between 6 and 13 $MPa^{1/2}$, more preferably between 7 and 13 $MPa^{1/2}$, better still between 7 and 12.5 $MPa^{1/2}$.

Preferably, these compounds are liquid at a temperature of 25°C and atmospheric pressure (760 mmHg).

The organic compound or compounds exhibiting a value for the Hansen solubility parameter δH as defined above are, for example, described in the reference work "Hansen solubility parameters: A user's handbook", Charles M. Hansen, CRC Press, 2000, pages 167 to 185, or else in the work "Handbook of Solubility Parameters and Other Cohesion Parameters", CRC Press, pages 95 to 121 and pages 177 to 185.

This value for the solubility parameter δH is related to the formation of hydrogen bonds.

In particular, the work "Handbook of Solubility Parameters and Other Cohesion Parameters", CRC Press, pages 95 to 121 and pages 177 to 185, gives the equation

$$\delta H = (\Sigma - {}^{z}U_h / V)^{1/2}$$

where

> ${}^{z}U_h$ (in $J.mol^{-1}$) describes the contributions of the functional group under consideration in the solubility parameters related to the hydrogen bonds (values in Table 14, page 183); this parameter ${}^{z}U_h$ is also described in the work "The relation between surface tension and solubility parameter in liquids", Bagda E., Farbe Lack, 84, 212, 1978;
> and V is the volume of the molecule.

The value for the solubility parameter δH is usually given for a temperature of 25°C and at atmospheric pressure (760 mmHg).

The organic compound or compounds (II) chosen from benzyl alcohol generally represent from 0.1 to 20% by weight, preferably from 1 to 5% by weight, of the total weight of the composition.

The composition according to the invention can also comprise one or more additional dyes. In particular, the composition according to the invention can also comprise at least one additional dye other than the acid dyes of abovementioned formula (1) to (6) chosen from natural dyes and nonnatural direct dyes, oxidation dye precursors or their combinations.

The term "natural dyes" is understood to mean any dye or dye precursor which occurs naturally and which is produced either by extraction (and optionally purification) from a plant matrix or by chemical synthesis.

The additional natural dyes suitable in particular for the implementation of the invention can be chosen, for example, from carminic acid, kermesic acid, isatin, chlorophyllins, hematein, hematoxylin, brazilin, brazilein, betanin,

flavonoids or anthocyanins.

Use may also be made of extracts or decoctions comprising these natural dyes and in particular henna-based extracts.

The composition can also comprise one or more additional nonnatural direct dyes other than the acid direct dyes described above chosen from ionic or nonionic entities, preferably cationic or nonionic entities.

Mention may be made, as examples of suitable additional direct dyes, of azo, methine, carbonyl, azine, nitro(hetero)aryl or tri(hetero)arylmethane direct dyes, porphyrins and phthalocyanines, alone or as mixtures.

More particularly, the azo dyes comprise an -N=N-functional group, the two nitrogen atoms of which are not simultaneously involved in a ring. However, it is not ruled out for one of the two nitrogen atoms of the -N=N- sequence to be involved in a ring.

The dyes of the family of the methines are more particularly compounds comprising at least one sequence chosen from >C=C< and -N=C<, the two atoms of which are not simultaneously involved in a ring. However, it is specified that one of the nitrogen or carbon atoms of the sequences can be involved in a ring. More particularly, the dyes of this family result from compounds of the following types: true methine (comprising one or more abovementioned -C=C- sequences); azomethine (comprising at least one or more-C=N- sequences) with, for example, the azacarbocyanines and their isomers, the diazacarbocyanines and their isomers, the tetraazacarbocyanines; mono- and diarylmethane; indoamines (or diphenylamines); indophenols; indoanilines.

As regards the dyes of the family of the carbonyls, mention may be made, for example, of nonnatural dyes chosen from acridone, benzoquinone, anthraquinone, naphthoquinone, benzanthrone, anthranthrone, pyranthrone, pyrazolanthrone, pyrimidinoanthrone, flavanthrone, indanthrone, flavone, (iso)violanthrone, isoindolinone, benzimidazolone, iso-quinolinone, anthrapyridone, pyrazoloquinazolone, perinone, quinacridone, quinophthalone, naphthalimide, anthrapyrimidine, diketopyrrolopyrrole or coumarin dyes.

As regards the dyes of the family of the cyclic azines, mention may in particular be made of azine, xanthene, thioxanthene, fluorindine, acridine, (di)oxazine, (di)thiazine or pyronine dyes.

The nitro(hetero)aromatic dyes are more particularly nitrobenzene or nitropyridine direct dyes.

As regards the dyes of porphyrin or phthalocyanine type, use may be made of cationic or noncationic compounds optionally comprising one or more metals or metal ions, such as, for example, alkali and alkaline earth metals, zinc and silicon.

Mention may be made, as examples of additional synthetic direct dyes which are particularly suitable, of nitrobenzene dyes, azo, azomethine or methine direct dyes, azacarbocyanines, such as tetraazacarbocyanines (tetraazapentamethines), quinone and in particular anthraquinone, naphthoquinone or benzoquinone direct dyes, or azine, xanthene, triarylmethane, indoamine, phthalocyanine and porphyrin direct dyes, alone or as mixtures. More preferably still, these additional direct dyes are chosen from nitrobenzene dyes, azo, azomethine or methine direct dyes and tetraazacarbocyanines (tetraazapentamethines); alone or as mixtures.

These dyes can be monochromophoric dyes (that is to say, comprising only a single chromophore) or polychromophoric dyes, preferably di- or trichromophoric dyes, it being possible for the chromophores to be identical or different and from the same or a different chemical family. It should be noted that a polychromophoric dye comprises several radicals, each resulting from a molecule which absorbs in the visible region between 400 and 800 nm. Furthermore, this absorbance of the dye requires neither preoxidation of the latter nor combination with other chemical entity(ies).

In the case of polychromophoric dyes, the chromophores are connected to one another by means of at least one connecting arm, which may or may not be cationic.

Mention may more particularly be made, among the polychromophoric dyes, of symmetrical or asymmetrical di- or trichromophoric azo and/or azomethine (hydrazone) dyes comprising, on the one hand, at least one optionally fused 5- or 6-membered aromatic heterocycle comprising at least one quaternized nitrogen atom participating in the said heterocycle and optionally at least one other heteroatom (such as nitrogen, sulphur or oxygen) and, on the other hand, at least one optionally substituted phenyl or naphthyl group optionally carrying at least one OR group with R representing a hydrogen atom, an optionally substituted $C_1$-$C_6$ alkyl radical or an optionally substituted phenyl ring or at least one $N(R')_2$ group with R', which are identical or different, representing a hydrogen atom, an optionally substituted $C_1$-$C_6$ alkyl radical or an optionally substituted phenyl ring, it being possible for the R' radicals to form, with the nitrogen atom to which they are bonded, a saturated 5- or 6-membered heterocycle, or alternatively either and/or both R' radicals can form, each with the carbon atom of the aromatic ring in the ortho position with respect to the nitrogen atom, a saturated 5- or 6-membered heterocycle.

Mention may preferably be made, as cationic aromatic heterocycle, of 5- or 6-membered rings comprising from 1 to 3 nitrogen atoms, preferably 1 or 2 nitrogen atoms, one being quaternized, the said heterocycle furthermore optionally being fused with a benzene ring. It should likewise be noted that the heterocycle can optionally comprise another heteroatom other than nitrogen, such as sulphur or oxygen.

If the heterocycles or phenyl or naphthyl groups are substituted, they are substituted, for example, by one or more $C_1$-$C_8$ alkyl radicals optionally substituted by a hydroxyl group, a $C_1$-$C_2$ alkoxy group, a $C_2$-$C_4$ hydroxyalkoxy group,

an acetylamino group, an amino group substituted by one or two $C_1$-$C_4$ alkyl radicals which optionally carry a hydroxyl group or it being possible for the two radicals to form, with the nitrogen atom to which they are attached, a 5- or 6-membered heterocycle optionally comprising another heteroatom identical to or different from nitrogen; a halogen atom; a hydroxyl group; a $C_1$-$C_2$ alkoxy radical; a $C_2$-$C_4$ hydroxyalkoxy radical; an amino radical; or an amino radical substituted by one or two identical or different $C_1$-$C_4$ alkyl radicals which optionally carry a hydroxyl group.

Preferably, the connecting arm is a linear, branched or cyclic $C_1$-$C_{20}$ alkyl chain; optionally interrupted by at least one heteroatom (such as nitrogen or oxygen) and/or by at least one group comprising it (CO, $SO_2$) ; optionally interrupted by at least one substituted or unsubstituted phenyl or naphthyl group; optionally interrupted by at least one saturated, unsaturated or aromatic heterocycle which may or may not be fused with a phenyl nucleus, the said heterocycle comprising at least one quaternized nitrogen atom participating in the said cycle and optionally at least one other heteroatom (such as oxygen, nitrogen or sulphur); optionally interrupted by at least one quaternary ammonium group substituted by two $C_1$-$C_{15}$ alkyl groups; the connecting arm not comprising a nitro, nitroso or peroxo group.

If the heterocycles or aromatic nuclei are substituted, they are substituted, for example, by one or more $C_1$-$C_8$ alkyl radicals optionally substituted by a hydroxyl group, a $C_1$-$C_2$ alkoxy group, a $C_2$-$C_4$ hydroxyalkoxy group, an acetylamino group, an amino group substituted by one or two identical or different $C_1$-$C_4$ alkyl radicals which optionally carry a hydroxyl group or it being possible for the two radicals to form, with the nitrogen atom to which they are attached, a 5- or 6-membered heterocycle optionally comprising another heteroatom identical to or different from nitrogen; a halogen atom; a hydroxyl group; a $C_1$-$C_2$ alkoxy radical; a $C_2$-$C_4$ hydroxyalkoxy radical; an amino radical; or an amino radical substituted by one or two identical or different $C_1$-$C_4$ alkyl radicals which optionally carry a hydroxyl group.

The connection between the connecting arm and each chromophore is generally made by means of a heteroatom substituting the phenyl or naphthyl nucleus or by means of the quaternized nitrogen atom of the cationic heterocycle.

Mention may be made, among the azo, azomethine, methine or tetraazapentamethine monochromophoric direct dyes which can be used according to the invention, of the cationic dyes described in Patent Applications WO 95/15144, WO 95/0177.2 and EP 714 954; FR 2 189 006, FR 2 285 851, FR-2 140 205, EP 1 378 544 and EP 1 674 073.

Thus, mention may very particularly be made of the cationic direct dyes corresponding to the following formulae:

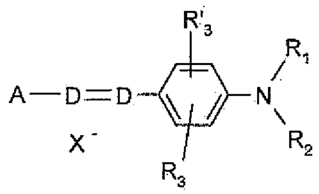

in which:

D represents a nitrogen atom or the -CH group,

$R_1$ and $R_2$, which are identical or different, represent a hydrogen atom; a $C_1$-$C_4$ alkyl radical which can be substituted by a -CN, -OH or -$NH_2$ radical or can form, with a carbon atom of the benzene ring, an optionally oxygen-comprising or nitrogen-comprising heterocycle which can be substituted by one or more $C_1$-$C_4$ alkyl radicals; or a 4'-aminophenyl radical,

$R_3$ and $R'_3$, which are identical or different, represent a hydrogen atom, a halogen atom chosen from chlorine, bromine, iodine and fluorine, a cyano radical, a $C_1$-$C_4$ alkyl radical, a $C_1$-$C_4$ alkoxy radical or an acetyloxy radical,

$X^-$ represents an anion, preferably chosen from chloride, methyl sulphate and acetate,

A represents a group chosen from the following structures:

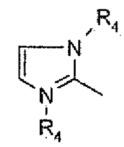 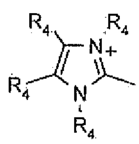 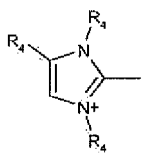 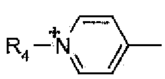 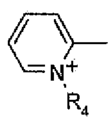

in which $R_4$ represents a $C_1$-$C_4$ alkyl radical which can be substituted by a hydroxyl radical;

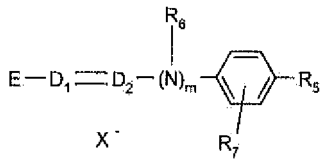

in which:

$R_5$ represents a hydrogen atom, a $C_1$-$C_4$ alkoxy radical or a halogen atom, such as bromine, chlorine, iodine or fluorine,
$R_6$ represents a hydrogen atom or a $C_1$-$C_4$ alkyl radical or forms, with a carbon atom in the benzene ring, a heterocycle which optionally comprises oxygen and/or is optionally substituted by one or more $C_1$-$C_4$ alkyl groups,
$R_7$ represents a hydrogen atom or a halogen atom, such as bromine, chlorine, iodine or fluorine,
$D_1$ and $D_2$, which are identical or different, represent a nitrogen atom or the -CH group,
m = 0 or 1,
$X^-$ represents a cosmetically acceptable anion preferably chosen from chloride, methyl sulphate and acetate,
E represents a group chosen from the following structures:

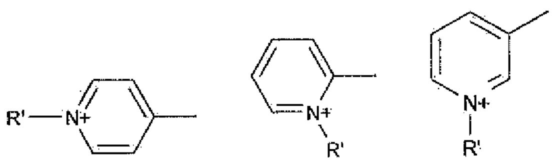

in which R' represents a $C_1$-$C_4$ alkyl radical;
when m = 0 and when $D_1$ represents a nitrogen atom, then E can also denote a group with the following structure:

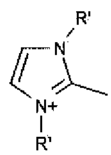

;

in which R' represents a $C_1$-$C_4$ alkyl radical.

Use is very particularly made, among the abovementioned compounds, of the following compounds:

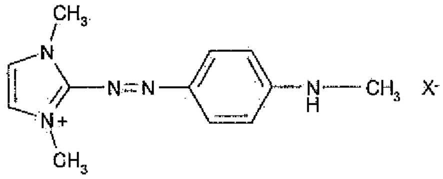

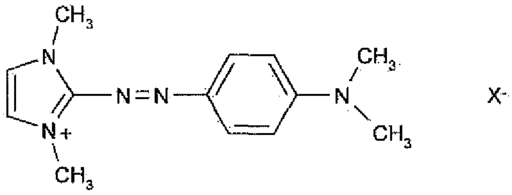

,

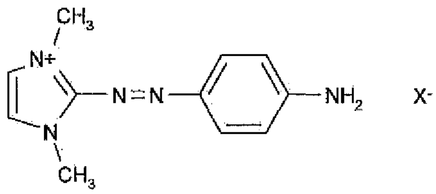

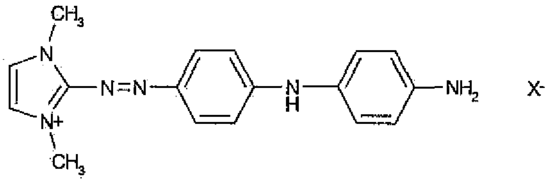

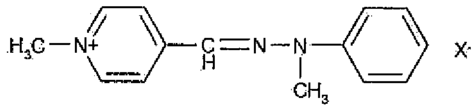

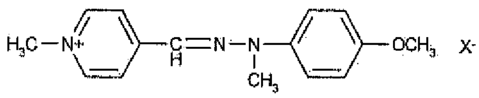

;

Mention may be made, among the dyes of tetraazapentamethine type which can be used according to the invention, of the following compounds appearing in the table below:

| | |
|---|---|
| 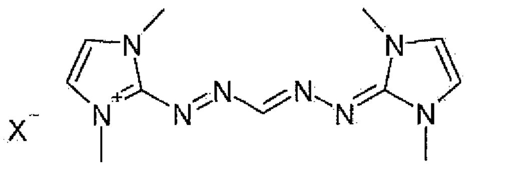 | 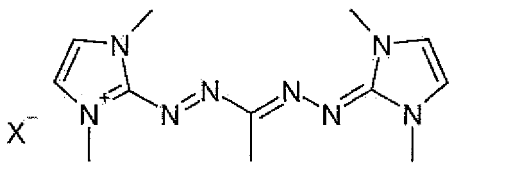 |
| 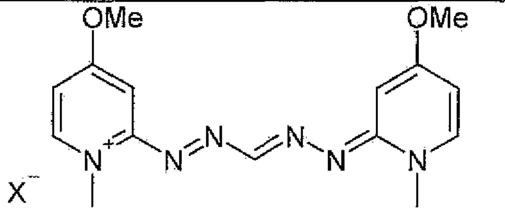 | 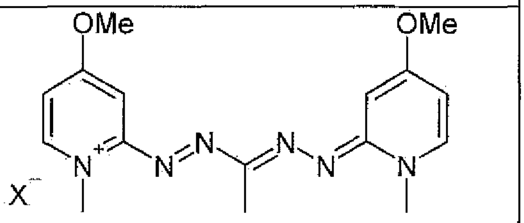 |
| 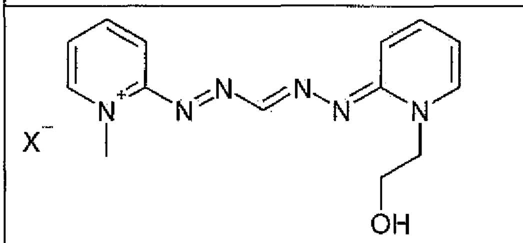 | 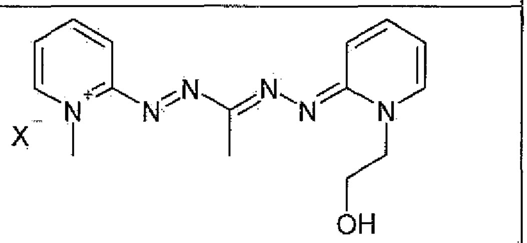 |
| 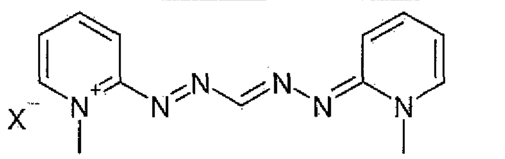 | 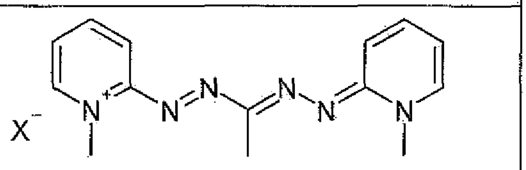 |
| 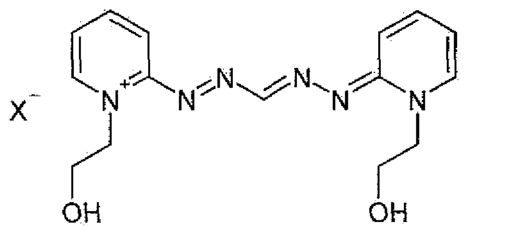 | 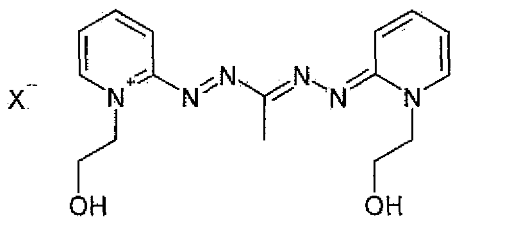 |

$X^-$ represents an anion preferably chosen from chloride, iodide, methyl sulphate, ethyl sulphate or acetate.

The dyeing composition can also comprise one or more oxidation dye precursors, more particularly one or more oxidation bases, optionally in combination with one or more couplers.

By way of example, the oxidation bases are chosen from para-phenylenediamines, bisphenylalkylenediamines, para-aminophenols, ortho-aminophenols, heterocyclic bases and their addition salts.

Mention may be made, among para-phenylenediamines, by way of example, of para-phenylenediamine, para-toluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-(β-hydroxyethyl)-para-phenylenediamine, 2-fluoro-para-phenylenedi amine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl, β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-(β-hydroxyethyloxy)-para-phenylenediamine, 2-(β-acetylaminoethyloxy)-para-phenylenediamine, N-(β-methoxyethyl)-para-phenylenediamine, 4-aminophenylpyrrolidine, 2-thienyl-para-phenylenediamine, 2-(β-hydroxyethylamino)-5-aminotoluene, 3-hydroxy-1-(4'-aminophenyl)pyrrolidine and their addition salts with an acid.

Among the para-phenylenediamines mentioned above, para-phenylenediamine, para-toluylenediamine, 2-isopropyl-para-phenylenediamine, 2-(β-hydroxyethyl)-para-phenylenediamine, 2-(β-hydroxyethyloxy)-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine, 2-(β-acetylaminoethyloxy)-para-phenylenediamine and their addition salts with an acid are particularly preferred.

Mention may be made, among bisphenylalkylenediamines, by way of example, of N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,6-dioxaoctane and their addition salts.

Mention may be made, among para-aminophenols, by way of example, of para-aminophenol, 4-amino-3-methylphenol, 4-amino-3-fluorophenol, 4-amino-3-chlorophenol, 4-amino-3-(hydroxymethyl)phenol, 4-amino-2-methylphenol, 4-amino-2-(hydroxymethyl)phenol, 4-amino-2-(methoxymethyl)phenol, 4-amino-2-(aminomethyl)phenol, 4-amino-2-[(β-hydroxyethyl)aminomethyl]phenol, 4-amino-2-fluorophenol and their addition salts with an acid.

Mention may be made, among ortho-aminophenols, by way of example, of 2-aminophenol, 2-amino-5-methylphenol, 2-amino-6-methylphenol, 5-acetamido-2-aminophenol and their addition salts.

Mention may be made, among heterocyclic bases, by way of example, of pyridine derivatives, pyrimidine derivatives and pyrazole derivatives.

Mention may be made, among pyridine derivatives, of the compounds described, for example, in Patents GB 1 026 978 and GB 1 153 196, such as 2,5-diaminopyridine, 2-[(4-methoxyphenyl)amino]-3-aminopyridine, 3,4-diaminopyridine and their addition salts.

Other pyridine oxidation bases of use in the present invention are the 3-aminopyrazolo[1,5-a]pyridine oxidation bases or their addition salts described, for example, in Patent Application FR 2 801 308. Mention may be made, by way of example, of pyrazolo[1,5-a]pyridin-3-ylamine; 2-(acetylamino)pyrazolo[1,5-a]pyridin-3-ylamine; 2-(morpholin-4-yl)pyrazolo[1,5-a]pyridin-3-ylamine; 3-aminopyrazolo-[1,5-a]pyridine-2-carboxylic acid; 2-methoxypyrazolo-[1,5-a]pyridin-3-ylamine; (3-aminopyrazolo[1,5-a]pyridin-7-yl)methanol; 2-(3-aminopyrazolo[1,5-a]pyridin-5-yl)ethanol; 2-(3-aminopyrazolo[1,5-a]pyridin-7-yl)ethanol; (3-aminopyrazolo[1,5-a]pyridin-2-yl)methanol; 3,6-diaminopyrazolo[1,5-a]pyridine; 3,4-diaminopyrazolo[1,5-a]pyridine; pyrazolo[1,5-a]pyridine-3,7-diamine; 7-(morpholin-4-yl)pyrazolo[1,5-a]pyridin-3-ylamine; pyrazolo[1,5-a]pyridine-3,5-diamine; 5-(morpholin-4-yl)pyrazolo[1,5-a]pyridin-3-ylamine; 2-[(3-aminopyrazolo[1,5-a]pyridin-5-yl)(2-hydroxyethyl)amino]ethanol; 2-[(3-aminopyrazolo[1,5-a]pyridin-7-yl)(2-hydroxyethyl)amino]ethanol; 3-amino-pyrazolo[1,5-a]pyridin-5-ol; 3-aminopyrazolo[1,5-a]pyridin-4-ol; 3-aminopyrazolo[1,5-a]pyridin-6-ol; 3-aminopyrazolo[1,5-a]pyridin-7-ol; and their addition salts.

Mention may be made, among pyrimidine derivatives, of the compounds described, for example, in Patents DE 2 359 399; JP 88-169571; JP 05-63124; EP 0 770 375 or Patent Application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine and their addition salts and their tautomeric forms, when a tautomeric equilibrium exists.

Mention may be made, among pyrazole derivatives, of the compounds described in Patents DE 3 843 892 and DE 4 133 957 and Patent Applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-(tert-butyl)-1-methylpyrazole, 4,5-diamino-1-(tert-butyl)-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-(hydroxymethyl)pyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-(methylamino)pyrazole, 3,5-diamino-4-(β-hydroxyethyl)amino-1-methylpyrazole and their addition salts. Use may also be made of 4,5-diamino-1-(β-methoxyethyl)pyrazole.

Mention may also be made, as pyrazole derivatives, of diamino-N,N-dihydropyrazolopyrazolones, in particular those described in Application FR 2 886 136, such as the following compounds and their addition salts: 2,3-diamino-6,7-dihydro-1H,5H-pyrazolo[1,2-a]pyrazol-1-one, 2-amino-3-ethylamino-6,7-dihydro-1H,5H-pyrazolo[1,2-a]pyrazol-1-one, 2-amino-3-isopropylamino-6,7-dihydro-1H,5H-pyrazolo[1,2-a]pyraxol-1-one, 2-amino-3-(pyrrolidin-1-yl)-6,7-dihydro-1H,5H-pyrazolo[1,2-alpyrazol-1-one, 4,5-diamino-1,2-dimethyl-1,2-dihydropyrazol-3-one, 4,5-diamino-1,2-diethyl-1,2-dihydropyrazol-3-one, 4,5-diamino-1,2-di(2-hydroxyethyl)-1,2-dihydropyrazol-3-one, 2-amino-3-(2-hydroxyethyl)amino-6,7-dihydro-1H,5H-pyrazolo[1,2-a]pyrazol-1-one, 2-amino-3-dimethylamino-6,7-dihydro-1H,5H-pyrazolo[1,2-a]pyrazol-1-one, 2,3-diamino-5,6,7,8-tetrahydro-1H,6H-pyridazino[1,2-a]pyrazol-1-one, 4-amino-1,2-diethyl-5-(pyrrolidin-1-yl)-1,2-dihydropyrazol-3-one, 4-amino-5-[3-(dimethylamino)pyrrolidin-1-yl]-1,2-diethyl-1,2-dihydropyrazol-3-one or 2,3-diamino-6-hydroxy-6,7-dihydro-1H,5H-pyrazolo[1,2-a]pyrazol-1-one.

Use will preferably be made, as heterocyclic bases, of 4,5-diamino-1-(β-hydroxyethyl)pyrazole and/or 2,3-diamino-6,7-dihydro-1H,5H-pyrazolo[1,2-a]pyrazol-1-one and their addition salts.

The composition according to the invention can optionally comprise one or more couplers advantageously chosen from those conventionally used for dyeing keratinous fibres.

Mention may in particular be made, among these couplers, of meta-phenylenediamines, meta-aminophenols, meta-diphenols, naphthalene couplers or heterocyclic couplers and their addition salts.

Mention may be made, by way of example, of 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene, 2,6-bis(β-hydroxyethylamino)toluene, 6-hydroxyindoline, 2,6-dihydroxy-4-methylpyridine, 1H-3-methylpyrazol-5-one, 1-phenyl-3-methylpyrazol-5-one, 2,6-dimethylpyrazolo[1,5-b][1,2,4]triazole, 2,6-dimethyl[3,2-c][1,2,4]triazole, 6-methylpyrazolo[1,5-a]benzimidazole, their addition salts with an acid and their mixtures.

Generally, the addition salts of the oxidation bases and couplers which can be used in the context of the invention are chosen in particular from the addition salts with an acid, such as hydrochlorides, hydrobromides, sulphates, citrates, succinates, tartrates, lactates, tosylates, benzenesulphonates, phosphates and acetates.

The oxidation base or bases, when they are present in the composition, advantageously represent from 0.0001 to 10% by weight, with respect to the weight of the composition, preferably from 0.005 to 5% by weight, with respect to the weight of the composition.

The coupler or couplers, if they are present, advantageously represent from 0.0001 to 10% by weight, with respect to the weight of the composition, preferably from 0.005 to 5% by weight, with respect to the weight of the composition.

When they are present, the additional dye or dyes represent from 0.01 to 10% by weight and preferably from 0.5 to 5% by weight, with respect to the weight of the composition.

The dyeing composition according to the invention can also comprise one or more conditioning agents.

Mention may be made, as examples, of volatile or nonvolatile and linear, cyclic, branched or unbranched silicones. These silicones can be provided in the form of oils, of resins or of gums; they can in particular be polyorganosiloxanes which are insoluble in the cosmetically acceptable medium.

Organopolysiloxanes are defined in more detail in the work by Walter Noll, "Chemistry and Technology of Silicones", (1968), Academic Press. They can be volatile or nonvolatile.

When they are volatile, the silicones are chosen more particularly from those having a boiling point of between 60°C and 260°C.

Use may also be made, as conditioning agent, of cationic polymers, such as polyquaterniums 22, 6, 10, 11, 35 and 37 and hexadimethrine chloride.

The concentration of conditioning agent(s) in the composition or compositions of use in the invention can vary from 0.01 to 10% by weight, with respect to the total weight of the composition, preferably from 0.05 to 5% by weight and more preferably still from 0.1 to 3% by weight.

The composition according to the invention can also comprise one or more organic thickening agents.

The organic thickening agents can be chosen from amides of fatty acids (coconut oil diethanolamide or monoethanolamide, oxyethylenated alkyl ether carboxylic acid monoethanolamide), polymeric thickeners, such as cellulose thickeners (hydroxyethylcellulose, hydroxypropylcellulose, carboxymethylcellulose), guar gum and its derivatives (hydroxypropyl guar), gums of microbial origin (xanthan gum, scleroglucan gum), crosslinked homopolymers of acrylic acid or of acrylamidopropanesulphonic acid and associative polymers (polymers comprising hydrophilic regions and hydrophobic regions having a fatty chain which are capable, in an aqueous medium, of reversibly associating with one another or with other molecules).

According to a specific embodiment, the thickener is polymeric and is chosen from cellulose thickeners (hydroxyethylcellulose, hydroxypropylcellulose, carboxymethylcellulose), guar gum and its derivatives (hydroxypropyl guar), gums of microbial origin (xanthan gum, scleroglucan gum) or crosslinked homopolymers of acrylic acid or of acrylamidopropanesulphonic acid.

As regards the associative thickening agents, use may be made of one or more polymers of nonionic or ionic nature, preferably anionic or cationic nature.

Their chemical structure more particularly comprises at least one hydrophilic region and at least one hydrophobic region. The term "hydrophobic group" is understood to mean a radical or polymer comprising a saturated or unsaturated and linear or branched hydrocarbon chain comprising at least 8 carbon atoms, preferably from 10 to 30 carbon atoms, in particular from 12 to 30 carbon atoms and more preferably from 18 to 30 carbon atoms.

Preferably, the hydrocarbon group originates from a monofunctional compound. By way of example, the hydrophobic group can result from a fatty alcohol, such as stearyl alcohol, dodecyl alcohol or decyl alcohol. It can also denote a hydrocarbon polymer, such as, for example, polybutadiene.

Mention may be made, among anionic amphiphilic polymers comprising at least one fatty (hydrophobic) chain, of:

- (I) the polymers comprising at least one hydrophilic unit and at least one allyl ether unit comprising a fatty chain, more particularly those for which the hydrophilic unit is composed of an ethylenic unsaturated anionic monomer, advantageously of a vinylcarboxylic acid and very particularly of an acrylic acid or a methacrylic acid or the mixtures of these, and for which the allyl ether unit comprising a fatty chain corresponds to the monomer of following formula (A):

$$CH_2 = C\ R'\ CH_2\ O\ B_n\ R \qquad (A)$$

in which R' denotes H or $CH_3$, B denotes the ethyleneoxy radical, n is zero or denotes an integer ranging from 1 to 100, and R denotes a hydrocarbon radical chosen from alkyl, arylalkyl, aryl, alkylaryl or cycloalkyl radicals comprising from 8 to 30 carbon atoms, preferably from 10 to 24 carbon atoms, and more particularly still from 12 to 18 carbon atoms. A more particularly preferred unit of formula (A) is a unit in which R' denotes H, n is equal to 10 and R denotes a stearyl ($C_{18}$) radical.

Preference is given, among these anionic polymers comprising a fatty chain, to the polymers formed from 20 to 60% by weight of acrylic acid and/or methacrylic acid, from 5 to 60% by weight of lower alkyl (meth)acrylates, from 2 to 50% by weight of allyl ether comprising a fatty chain of formula (A), and from 0 to 1% by weight of a crosslinking agent which is a well-known copolymerizable polyethylenic unsaturated monomer, such as diallyl phthalate, allyl (meth) acrylate, divinylbenzene, (poly) ethylene glycol dimethacrylate and methylenebisacrylamide.

Among the latter, preference is very particularly given to crosslinked terpolymers of methacrylic acid, of ethyl acrylate and of polyethylene glycol (10 EO) ether of stearyl alcohol (Steareth 10), in particular those sold by Allied Colloids under the names Salcare SC 80 and Salcare SC 90, which are 30% aqueous emulsions of a crosslinked terpolymer of methacrylic acid, of ethyl acrylate and of steareth-10 allyl ether (40/50/10).

- (II) the polymers comprising at least one hydrophilic unit of olefinic unsaturated carboxylic acid type and at least one hydrophobic unit of alkyl ($C_{10}$-$C_{30}$) ester of unsaturated carboxylic acid type.

Preferably, these polymers are chosen from those for which the hydrophilic unit of olefinic unsaturated carboxylic acid type corresponds to the monomer of following formula (B):

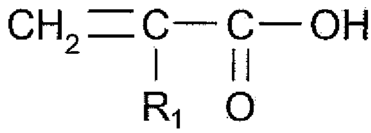

(B)

in which $R_1$ denotes H or $CH_3$ or $C_2H_5$, that is to say acrylic acid, methacrylic acid or ethacrylic acid units, and for which the hydrophobic unit of alkyl ($C_{10}$-$C_{30}$) ester of unsaturated carboxylic acid type corresponds to the monomer of following formula (C) :

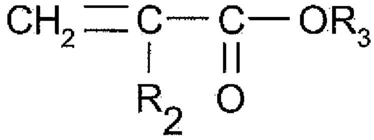

(C)

in which $R_2$ denotes H or $CH_3$ or $C_2H_5$ (that is to say, acrylate, methacrylate or ethacrylate units) and preferably H (acrylate units) or $CH_3$ (methacrylate units), $R_3$ denoting a $C_{10}$-$C_{30}$ and preferably $C_{12}$-$C_{22}$ alkyl radical.

The alkyl ($C_{10}$-$C_{30}$) esters of unsaturated carboxylic acids are, for example, lauryl acrylate, stearyl acrylate, decyl acrylate, isodecyl acrylate, dodecyl acrylate, and the corresponding methacrylates, lauryl methacrylate, stearyl methacrylate, decyl methacrylate, isodecyl methacrylate and dodecyl methacrylate.

Use will more particularly be made, among anionic polymers comprising a fatty chain of this type, of polymers formed from a mixture of monomers comprising:

(i) essentially acrylic acid,
(ii) an ester of formula (C) described above and in which $R_2$ denotes H or $CH_3$, $R_3$ denoting an alkyl radical having from 12 to 22 carbon atoms, and
(iii) a crosslinking agent which is a well-known copolymerizable polyethylenic unsaturated monomer, such as diallyl phthalate, allyl (meth)acrylate, divinylbenzene, (poly)ethylene glycol dimethacrylate and methylenebisacrylamide.

Use will more particularly be made, among anionic polymers comprising a fatty chain of this type, of those composed of 95 to 60% by weight of acrylic acid (hydrophilic unit), 4 to 40% by weight of $C_{10}$-$C_{30}$ alkyl acrylate (hydrophobic unit) and 0 to 6% by weight of crosslinking polymerizable monomer or else those composed of 98 to 96% by weight of acrylic acid (hydrophilic unit), 1 to 4% by weight of $C_{10}$-$C_{30}$ alkyl acrylate (hydrophobic unit) and 0.1 to 0.6% by weight of crosslinking polymerizable monomer such as those described above.

Preference is very particularly given according to the present invention, among the said polymers above, to the products sold by Goodrich under the trade names Pemulen TR1, Pemulen TR2 or Carbopol 1382 and more preferably still to Pemulen TR1 and the product sold by S.E.P.P.I.C. under the name Coatex SX.

-(III) maleic anhydride/$C_{30}$-$C_{38}$ $\alpha$-olefin/alkyl maleate terpolymers, such as the product (maleic anhydride/$C_{30}$-$C_{38}$ $\alpha$-olefin/isopropyl maleate copolymer) sold under the name Performa V 1608 by Newphase Technologies.

-(IV) acrylic terpolymers comprising:

(a) 20% to 70% by weight of a carboxylic acid comprising $\alpha,\beta$-monoethylenic unsaturation,

(b) 20 to 80% by weight of a non-surface-active monomer comprising $\alpha,\beta$-monoethylenic unsaturation other than (a),

(c) 0.5 to 60% by weight of a nonionic monourethane which is the reaction product of a monohydric surfactant with a monoisocyanate comprising monoethylenic unsaturation,

such as those described in Patent Application EP-A-0 173 109 and more particularly a methacrylic acid/methyl acrylate/dimethyl(meta-isopropenyl)benzyl isocyanate of ethoxylated behenyl alcohol (40 EO) terpolymer as a 25% aqueous dispersion.

- (V) copolymers comprising, among their monomers, a carboxylic acid comprising $\alpha,\beta$-monoethylenic unsaturation and an ester of a carboxylic acid comprising $\alpha,\beta$-monoethylenic unsaturation and of an oxyalkylenated ($C_8$-$C_{30}$) fatty alcohol.

Preferably, these compounds also comprise, as monomer, an ester of a carboxylic acid comprising $\alpha,\beta$-monoethylenic unsaturation and of a $C_1$-$C_4$ alcohol.

Mention may be made, as an example of this type of compound, of Aculyn 22, sold by Röhm & Haas, which is a methacrylic acid/ethyl acrylate/oxyalkylenated stearyl methacrylate terpolymer.

The nonionic amphiphilic polymers comprising a fatty (hydrophobic) chain are preferably chosen from:

- (1) celluloses modified by groups comprising at least one fatty chain, such as, in particular;
    - hydroxyethylcelluloses modified by groups comprising at least one fatty chain, such as alkyl, arylalkyl or alkylaryl groups, or their mixtures, and in which the alkyl groups are preferably $C_8$-$C_{22}$ alkyl groups, such as the product Natrosol Plus Grade 330 CS ($C_{16}$ alkyl) sold by Aqualon or the product Bermocoll EHM 100 sold by Berol Nobel,
    - those modified by alkylphenol polyalkylene glycol ether groups, such as the product Amercell Polymer HM-1500 (nonylphenol polyethylene glycol (15) ether) sold by Amerchol.
- (2) hydroxypropyl guars modified by groups comprising at least one fatty chain, such as the product Esaflor HM 22 ($C_{22}$ alkyl chain) sold by Lamberti or the products RE210-18 ($C_{14}$ alkyl chain) and RE205-1 ($C_{20}$ alkyl chain) sold by Rhône-Poulenc.
- (3) copolymers of vinylpyrrolidone and of hydrophobic monomers comprising a fatty chain, with, for example:
    - the products Antaron V216 or Ganex V216 (vinylpyrrolidone/hexadecene copolymer) sold by I.S.P.,
    - the products Antaron V220 or Ganex V220 (vinylpyrrolidone/eicosene copolymer) sold by I.S.P.
- (4) copolymers of $C_1$-$C_6$ alkyl methacrylates or acrylates and of amphiphilic monomers comprising at least one fatty chain, such as, for example, the methyl acrylate/oxyethylenated stearyl acrylate copolymer sold by Goldschmidt under the name Antil 208.
- (5) copolymers of hydrophilic methacrylates or acrylates and of hydrophobic monomers comprising at least one fatty chain, such as, for example, the polyethylene glycol methacrylate/lauryl methacrylate copolymer.
- (6) polymers comprising an aminoplast ether backbone having at least one fatty chain, such as the compounds Pure Thix provided by Sud-Chemie.
- (7) polyether polyurethanes comprising, in their chain, both hydrophilic sequences of generally polyoxyethylene nature and hydrophobic sequences which can be aliphatic strings only and/or cycloaliphatic and/or aromatic strings.

Preferably, the polyether polyurethanes comprise at least two hydrocarbon fatty chains having from 8 to 30 carbon atoms which are separated by a hydrophilic sequence, it being possible for the hydrocarbon chains to be pendant chains or chains at the end of the hydrophilic sequence.

Use may be made, as examples of nonionic polyether polyurethanes comprising a fatty chain which can be used in the invention, of Rheolate 205 comprising a urea functional group sold by Rheox or else Rheolates 208, 204 or 212, and also Acrysol RM 184, Aculyn or Acrysol 44 and Aculyn or Acrysol 46 from Röhm & Haas [Aculyn 46 is a polycondensate of polyethylene glycol comprising 150 or 180 mol of ethylene oxide, of stearyl alcohol and of methylenebis(4-cyclohexyl isocyanate) (SMDI), at 15% by weight in a matrix of maltodextrin (4%) and of water (81%); Aculyn 44 is a polycondensate of polyethylene glycol comprising 150 or 180 mol of ethylene oxide, of decyl alcohol and of methylenebis-(4-cyclohexyl isocyanate) (SMDI), at 35% by weight in a mixture of propylene glycol (39%) and of water (26%)].

Mention may also be made of the product Elfacos T210 comprising a $C_{12}$-$C_{14}$ alkyl chain and the product Elfacos T212 comprising a $C_{18}$ alkyl chain from Akzo, and also the product DW 1206B from Röhm & Haas comprising a $C_{20}$ alkyl chain and comprising a urethane bond, provided at a dry matter content of 20% in water.

Use may also be made of solutions or dispersions of these polymers, in particular in water or in an aqueous/alcoholic medium. Mention may be made, as examples of such polymers, of Rheolate 255, Rheolate 278 and Rheolate 244, sold by Rheox. Use may also be made of the products DW 1206F and DW 1206J provided by Röhm & Haas.

The polyether polyurethanes which can be used according to the invention are in particular those described in the paper by G. Fonnum J. Bakke and Fk. Hansen, Colloid Polym. Sci., 271, 380-389 (1993).

The cationic amphiphilic polymers comprising at least one fatty (hydrophobic) chain used can be chosen in particular from quaternized cellulose derivatives, cationic polyurethanes or cationic polyvinyllactams and preferably from quaternized cellulose derivatives.

Mention may in particular be made, as examples of polymers of this type, of:

- quaternized celluloses modified by groups comprising at least one fatty chain, such as alkyl, arylalkyl or alkylaryl groups comprising at least 8 carbon atoms, or mixtures of these,
- quaternized hydroxyethylcelluloses modified by groups comprising at least one fatty chain, such as alkyl, arylalkyl, or alkylaryl groups comprising at least 8 carbon atoms, or mixtures of these.

The alkyl radicals carried by the quaternized celluloses or hydroxyethylcelluloses above preferably comprise from 8 to 30 carbon atoms. The aryl radicals preferably denote the phenyl, benzyl, naphthyl or anthryl groups.

Examples of quaternized alkylhydroxyethylcelluloses comprising $C_8$-$C_{30}$ fatty chains which may be indicated are the products Quatrisoft LM 200, Quatrisoft LM-X 529-18-A, Quatrisoft LM-X 529-18B ($C_{12}$ alkyl) and Quatrisoft LM-X 529-8 ($C_{18}$ alkyl) sold by Amerchol and the products Crodacel QM, Crodacel QL ($C_{12}$ alkyl) and Crodacel QS ($C_{18}$ alkyl) sold by Croda.

The content of thickening polymers, if they are present, usually varies from 0.05% to 5% by weight, with respect to the weight of the dyeing composition.

According to a particularly advantageous embodiment, the dyeing composition according to the invention comprises one or more surface-active agents. The latter can be chosen without distinction, alone or as mixtures, from anionic, amphoteric, nonionic or zwitterionic and cationic surfactants.

As regards the anionic surfactants, use is usually made of the salts, in particular the alkali metal salts, such as sodium salts, ammonium salts, amine salts, aminoalcohol salts or alkaline earth metal salts, for example magnesium salts, of the following compounds, alone or as mixtures:

- alkyl sulphates, alkyl ether sulphates, alkylamido ether sulphates or alkylaryl polyether sulphates;
- alkylsulphonates, alkylamidesulphonates or alkyl-arylsulphonates;
- alkyl sulphosuccinates, alkyl ether sulphosuccinates or alkylamide sulphosuccinates;
- alkyl sulphoacetates;
- acylsarcosinates; and acylglutamates;
- alkyl esters of polyglycosidecarboxylic acids, such as alkyl glucosidecitrates, alkyl polyglycosidetartrates and alkyl polyglycosidesulphosuccinates;
- alkyl sulphosuccinamates;
- acylisethionates, N-acyltaurates or acyllactylates;
- alkyl-D-galactosideuronic acids;
- polyoxyalkylenated alkyl ether carboxylic acids, polyoxyalkylenated alkylaryl ether carboxylic acids or polyoxyalkylenated alkylamido ether carboxylic acids;
- the alkyl or acyl (RCO-) group of these compounds comprising from 10 to 24 carbon atoms and the aryl group preferably denoting a phenyl or benzyl group; the number of oxyalkylene groups, preferably oxyethylene groups, is

between 2 and 50.

As regards the nonionic surfactants, the latter can advantageously be chosen from the following compounds, alone or as mixtures:

- polyethoxylated, polypropoxylated or polyglycerolated fatty alcohols,
- polyethoxylated, polypropoxylated or polyglycerolated $\alpha$-diols,
- the number of ethylene oxide or propylene oxide groups ranging from 2 to 50; the number of glycerol groups ranging from 2 to 30;
- condensates of ethylene oxide and of propylene oxide with fatty alcohols;
- polyethoxylated fatty amides having from 2 to 30 mol of ethylene oxide;
- polyglycerolated fatty amides comprising from 1 to 5 glycerol groups;
- ethoxylated esters of fatty acids and of sorbitan having 2 to 30 mol of ethylene oxide, or esters of fatty acids and of sucrose;
- alkylpolyglucosides or N-alkylglucamine derivatives; these compounds comprising at least one alkyl or alkenyl chain comprising from 10 to 24 carbon atoms;
- copolymers of ethylene oxide and of propylene oxide.

The cationic surfactants participating in the composition according to the invention can be chosen in particular from the following compounds, alone or as mixtures:

- primary, secondary or tertiary fatty amines which are optionally polyethoxylated (2 to 30 mol of ethylene oxide) and their salts,
- quaternary ammonium salts, such as tetraalkylammonium, alkylamidoalkyltrialkylammonium, trialkylbenzylammonium, trialkylhydroxyalkylammonium or alkylpyridinium chlorides or bromides,
- alkylimidazoline derivatives;

these compounds comprising at least one alkyl chain comprising from 10 to 24 carbon atoms.

Finally, the amphoteric surfactants can be chosen from the following compounds, alone or as mixtures:

- derivatives of secondary or tertiary aliphatic amines in which the aliphatic group is a linear or branched chain comprising from 10 to 24 carbon atoms and comprising at least one water-solubilizing anionic group, such as, for example, a carboxylate, sulphonate, sulphate, phosphate or phosphonate group,
- alkyl betaines, sulphobetaines, alkyl amido ($C_6$-$C_8$) alkyl betaines or alkyl amido ($C_6$-$C_8$) alkyl sulphobetaines;

these compounds comprising at least one alkyl chain comprising from 10 to 24 carbon atoms.

Preferably, the surfactants are nonionic, anionic or amphoteric and more preferably still nonionic.

Usually, the surface-active agents represent from 0.01 to 50% by weight, preferably from 0.1 to 25% by weight, with respect to the weight of the composition.

The dyeing composition according to the invention can also comprise various adjuvants conventionally used in hair dyeing compositions, such as, for example, cationic, anionic, nonionic, amphoteric or zwitterionic polymers other than the thickeners mentioned above, or their mixtures; inorganic thickening agents, such as, in particular, clays; antioxidants, such as, for example, ascorbic acid or erythorbic acid; reducing agents other than the compounds (II) mentioned above with, inter alia, ammonium sulphite, bisulphite or metabisulphite or ammonium thiolactate; penetration agents; sequestering agents, such as ethylenediaminetetraacetic acid or its salts; fragrances; matifying agents with, for example, titanium oxide; buffers; dispersing agents; film-forming agents; ceramides and preservatives.

The above adjuvants are generally present in an amount of, for each of them, between 0.01 and 20% by weight, with respect to the weight of the composition.

The composition according to the invention can also comprise a cosmetically acceptable medium.

The cosmetically acceptable medium of the composition, which is a medium appropriate for dyeing human keratinous fibres, preferably comprises water and optionally one or more additional solvents other than the organic compounds (II) exhibiting a value for the Hansen solubility parameter $\delta H$ of between 8 and 15 $MPa^{1/2}$ and with a molecular weight of less than 250 g/mol mentioned above.

Mention may be made, for example, as examples of such additional solvents, of linear or branched $C_2$-$C_4$ alkanols, such as ethanol and isopropanol; polyols and polyol ethers, such as 2-butoxyethanol, hexylene glycol, propylene glycol, dipropylene glycol or glycerol, and their mixtures.

The additional solvent or solvents other than the organic compounds defined above present in the composition can be present in proportions preferably ranging from 1 to 40% by weight, with respect to the total weight of the dyeing

composition, more preferably still from 5 to 30% by weight.

The amount of water in the composition according to the invention is preferably greater than 10% by weight, with respect to the weight of the composition, more advantageously greater than or equal to 25% by weight. Preferably, the water content is between 25 and 98% by weight, with respect to the weight of the composition.

The pH of the composition according to the invention is preferably less than or equal to, preferably less than, 7. In particular, the pH is between 2 and 6, limits included, advantageously between 2 and 4, limits included.

It can be adjusted to the desired value using one or more acidifying agents or one or more basifying agents normally used in the field.

Mention may be made, among acidifying agents, as examples, of inorganic or organic acids, such as hydrochloric acid, orthophosphoric acid, sulphuric acid, carboxylic acids, such as acetic acid, tartaric acid, citric acid or lactic acid, or sulphonic acids.

Mention may be made, among basifying agents, as examples, of alkaline carbonates, alkanolamines, such as mono-, di- and triethanolamines and their derivatives, sodium hydroxide, potassium hydroxide and of compounds of following formula:

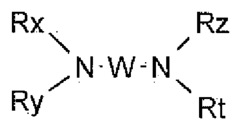

in which W is a propylene residue optionally substituted by a hydroxyl group or a $C_1$-$C_6$ alkyl radical and Rx, Ry, Rz and Rt, which are identical or different, represent a hydrogen atom, a $C_1$-$C_6$ alkyl radical or a $C_1$-$C_6$ hydroxyalkyl radical.

The composition according to the invention can also comprise one or more oxidizing agents.

In particular, the composition of oxidizing agent is obtained by mixing at the time of use, before application, a composition described above with at least one composition comprising one or more oxidizing agents.

The oxidizing agent is preferably chosen from hydrogen peroxide, urea hydrogen peroxide, alkali metal bromates or ferricyanides, or peroxygenated salts, such as, for example, persulphates, perborates or percarbonates of alkali metals or alkaline earth metals, such as sodium, potassium or magnesium.

The use of hydrogen peroxide is particularly preferred.

This oxidizing agent is advantageously composed of hydrogen peroxide in aqueous solution (aqueous hydrogen peroxide solution), the assay of which can vary more particularly from 1 to 40 volumes and more preferably still from 5 to 40 volumes.

The compositions according to the invention can result from mixing at the time of use of several compositions.

Another subject-matter of the invention is thus composed of a method for dyeing keratinous fibres, in particular human keratinous fibres, such as the hair, which consists in applying the composition according to the invention described above.

In accordance with a first embodiment, the composition applied does not comprise an oxidizing agent. This embodiment is appropriate in particular in the case where the composition does not comprise an oxidation dye precursor (bases, couplers).

In accordance with a second embodiment, the composition is applied in the presence of at least one oxidizing agent.

This embodiment can be employed if the composition comprises, as dyes, only direct dyes (acid direct dye(s) and optionally one or more additional direct dyes) or alternatively if the composition comprises one or more acid direct dyes and optionally one or more additional direct dyes, in combination with one or more oxidation dye precursors (bases and couplers).

According to a first alternative form of this second embodiment, the composition which has just been explained in detail and which is obtained by mixing at the time of use, before application, a composition according to the invention devoid of oxidizing agent with an oxidizing composition is applied to the fibres.

According to a second alternative form of this second embodiment, the composition according to the invention devoid of oxidizing agent and an oxidizing composition are applied successively and without intermediate rinsing.

The oxidizing composition employed comprises one or more oxidizing agents as defined above.

As regards the organic solvents optionally present in the oxidizing composition, reference may be made to the list indicated above in the context of the specifications of the composition according to the invention. These organic solvents can also be chosen from the organic compounds (II) chosen from benzyl alcohol present in the composition according to the invention exhibiting a value for the Hansen solubility parameter $\delta H$ of between 5 and 16 $MPa^{1/2}$ and with a molecular weight of less than 250 g/mol.

The oxidizing composition can be provided in the form of a solution, an emulsion or a gel.

It can optionally comprise one or more additives conventionally used in the field of the dyeing of human keratinous

fibres, according to the formulation form desired. Reference may here again be made to the list of the additives given above.

Whatever the embodiment selected (with or without oxidizing agent), the mixture applied to the fibres is left in place for a period of time, generally, of the order of 1 minute to 1 hour, preferably of 10 minutes to 30 minutes.

The temperature during the method is conventionally between 10 and 200°C and more particularly between ambient temperature (between 15 and 25°C) and 80°C, preferably between ambient temperature and 60°C.

On conclusion of the treatment, the human keratinous fibres are optionally rinsed with water, washed with a shampoo, again rinsed with water and then dried or left to dry.

The invention is illustrated by the following examples.

Another subject-matter of the present invention is a multicompartment device in which one of the compartments includes the composition according to the invention, devoid of oxidizing agent and comprising one or more acid direct dyes of formula (1) to (6) and one or more organic compounds (II) chosen from benzyl alcohol, and a second compartment includes an oxidizing composition.

**Example 1**

The following dyeing compositions are prepared from the following ingredients in the following proportions shown in g per 100 g of composition:

Dyeing gel

| | **Comparative Composition A** | **Inventive Composition B** |
|---|---|---|
| Indigo carmine [1] | 0.5 g | 0.5 g |
| Ethanol | 16 g | 10 g |
| Propylene glycol butyl ether | - | 6 g |
| pH agent | q.s. for pH = 2.7 | q.s. for pH = 2.7 |
| Fragrance | q.s. | q.s. |
| Water | q.s. for 100 g | q.s. for 100 g |
| [1] Indigo carmine, CAS = 860-22-0 | | |

Pairs of locks of hair comprising 90% of natural white hairs are dyed at 40°C for 20 minutes with these dyeing compositions A and B and the locks are subsequently superficially dried, rinsed, shampooed and dried.

The dyeing results on locks are observed visually and are accompanied by colorimetric measurements of absorbances of the colour expressed in ΔE with respect to the undyed hair.

It was found that the composition B of the invention makes it possible very markedly to improve the colour on lock with respect to a composition of the prior art (composition A).

These results are confirmed by the colorimetric measurements, which indicate significant increases in absorbance of colour with the composition B of the invention.

| | Absorbance on natural hair | | | |
|---|---|---|---|---|
| | L* | a* | b* | ΔE/undyed hair |
| Undyed hair | 59.28 | 0.62 | 12.63 | - |
| Hair dyed by A | 40.09 | -12.08 | -10.07 | 32.32 |
| Hair dyed by B | 37.59 | -13.99 | -14.51 | 37.70 |

A better intensity of the colour, reflected by a significantly lower L* for the colouring obtained with the composition B of the invention, is also found.

**Example 2**

The following dyeing compositions are prepared from the following ingredients in the following proportions shown in g per 100 g of composition:

Dyeing gel

| | Composition C | Composition D | Composition E |
|---|---|---|---|
| Indigo carmine [1] | 0.5 g | 0.5 g | 0.5 g |
| Ethanol | 15 g | 16 g | 15 g |
| Benzyl alcohol | 5 g | 1 g | - |
| 3-Phenyl-1-propanol | - | 0.5 g | - |
| 2-Phenyl-1-ethanol | - | 1 g | - |
| Benzoic acid | 0.5 g | 0.5 g | |
| Decanol | - | - | 5 g |
| Sodium lauryl sulphate | - | - | 2 g |
| pH agent | q.s. for pH = 2.7 | q.s. for pH = 2.7 | q.s. for pH = 2.7 |
| Fragrance | q.s. | q.s. | q.s. |
| Water | q.s. for 100 g | q.s. for 100 g | q.s. for 100 g |
| [1] Indigo carmine, CAS = 860-22-0 | | | |

Pairs of locks of hair comprising 90% of natural white hairs which have been permed are dyed at 40°C for 20 minutes with these dyeing compositions C to E and the locks are subsequently superficially dried, rinsed, shampooed and dried.

Powerful blue colourings which are resistant to shampooing operations and to UV radiation are obtained.

**Example 3**

The following dyeing compositions are prepared from the following ingredients in the following proportions shown in g per 100 g of composition:

Dyeing gel

| | Composition F | Composition G | Composition H |
|---|---|---|---|
| Indigo carmine [1] | 0.2 g | 0.2 g | 0.2 g |
| Curcumin | 0.5 g | 0.5 g | 0.5 g |
| Isatin | 0.3 g | 0.3 g | 0.3 g |
| Orcein | 0.3 g | 0.3 g | 0.3 g |
| Chlorophyllin | 0.15 g | 0.15 g | 0.15 g |
| Sorghum | 0.02 g | 0.02 g | 0.02 g |
| Laccaic acid | 0.01 g | 0.01 g | 0.01 g |
| Ethanol | 15 g | 16 g | 15 g |
| Benzyl alcohol | 5 g | 1 g | - |
| 3-Phenyl-1-propanol | - | 0.5 g | - |
| 2-Phonyl-1-ethanol | - | 1 g | - |
| Benzoic acid | 0.5 g | 0.5 g | - |
| Decanol | - | - | 5 g |
| Sodium lauryl sulphate | - | - | 2 g |
| pH agent | q.s. for pH = 2.7 | q.s. for pH = 2.7 | q.s. for pH = 2.7 |
| Fragrance | q.s. | q.s. | q.s. |

(continued)

| | Composition F | Composition G | Composition H |
|---|---|---|---|
| Water | q.s. for 100 g | q.s. for 100 g | q.s. for 100 g |
| [1] Indigo carmine, CAS = 860-22-0 | | | |

Pairs of locks of hair comprising 90% of natural white hairs which have been permed are dyed at 40°C for 20 minutes with these dyeing compositions F to G and the locks are subsequently superficially dried, rinsed, shampooed and dried.

Powerful brown colourings which are resistant to shampooing operations and to UV radiation are obtained.

## Claims

1. Dyeing composition for dyeing human keratinous fibres comprising:

- (a) one or more acid direct dyes chosen from the following compounds and their mixtures:

| | |
|---|---|
| 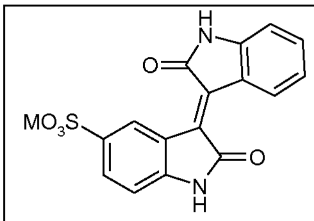 (1) | 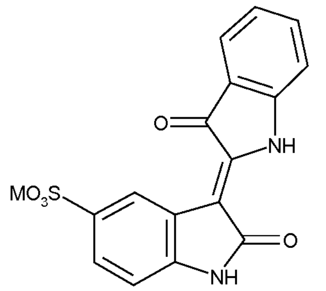 (2) |
| 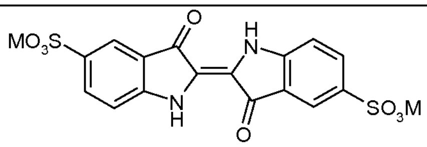 (3) | 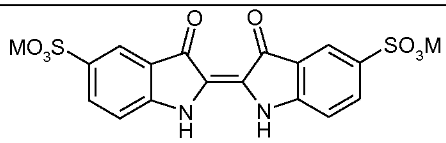 (4) |
| 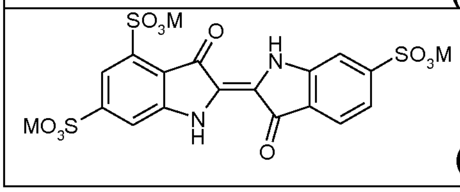 (5) | 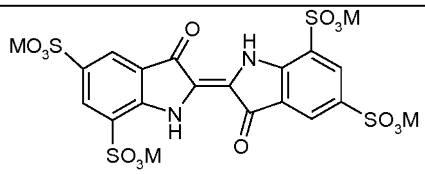 (6) |

M representing a hydrogen atom, a sodium ion or a potassium ion, the content of this (these) acid direct dye(s) being at least 0.01% by weight, with respect to the weight of the composition; and

- (b) one or more organic compounds (II) exhibiting a value for the Hansen solubility parameter $\delta H$ of between 5 and 16 $MPa^{1/2}$, as defined in the "Handbook of Solubility Parameters and Other Cohesion Parameters", CRC Press, pages 95 to 121 and pages 177 to 185, and with a molecular weight of less than 250 g/mol, at 25°C, said solvent being liquid at a temperature of 25°C and atmospheric pressure (760 mmHg), said compound being benzyl alcohol.

2. Composition according to the preceding claim, **characterized in that** the dye is the compound (3), more particularly in the sodium salt form.

3. Composition according to any one of the preceding claims, **characterized in that** the acid direct dye or dyes represent from 0.01 to 20% by weight, preferably from 0.05 to 10% by weight, better still from 0.1 to 5% by weight, of the total weight of the cosmetic composition.

4. Composition according to any one of the preceding claims, **characterized in that** the organic compound or compounds (II) represent from 0.1 to 20% by weight, preferably from 1 to 5% by weight, of the total weight of the composition.

5. Composition according to any one of the preceding claims, **characterized in that** the composition comprises one or more additional dyes other than the acid direct dyes chosen from natural dyes and non-natural direct dyes, oxidation dye precursors or their combinations.

6. Composition according to any one of the preceding claims, **characterized in that** it comprises water and optionally one or more additional organic solvents other than the said organic compounds (II).

7. Composition according to any one of the preceding claims, **characterized in that** it comprises one or more oxidizing agents, preferably hydrogen peroxide.

8. Dyeing method which consists in applying, to keratinous fibres, in particular human keratinous fibres, such as the hair, a composition as defined in any one of the preceding claims.

9. Multicompartment device in which one of the compartments includes a composition according to any one of Claims 1 to 6 and a second compartment includes an oxidizing composition.

**Patentansprüche**

1. Färbezusammensetzung zum Färben von menschlichen Keratinfasern, umfassend:

   - (a) einen oder mehrere saure Direktfarbstoffe, die aus den folgenden Verbindungen und Mischungen davon ausgewählt sind:

| | |
|---|---|
| 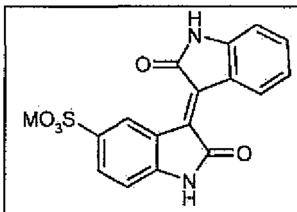 (1) | 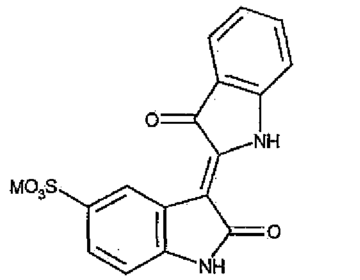 (2) |
| 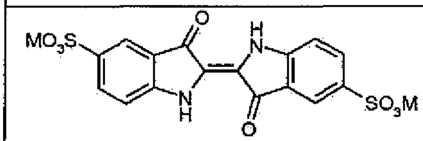 (3) | 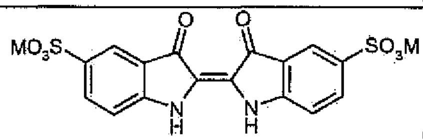 (4) |
| 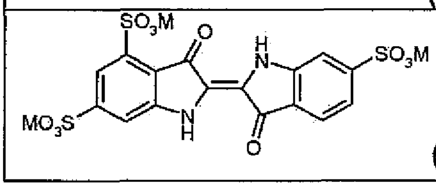 (5) | 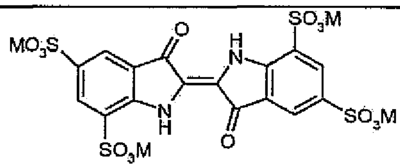 (6) |

   wobei M für ein Wasserstoffatom, ein Natriumion oder ein Kaliumion steht, wobei der Gehalt dieses sauren Direktfarbstoffs bzw. dieser sauren Direktfarbstoffe mindestens 0,01 Gew.-%, bezogen auf das Gewicht der Zusammensetzung, beträgt; und
   - (b) eine oder mehrere organische Verbindungen (II) mit einem Wert für den Hansen-Löslichkeitsparameter $\delta H$ zwischen 5 und 16 $MPa^{1/2}$ gemäß der Definition im "Handbook of Solubility Parameters and Other Cohesion Parameters", CRC Press, Seiten 95 bis 121 und Seiten 177 bis 185, und mit einem Molekulargewicht von weniger als 250 g/mol bei 25 °C, wobei das Lösungsmittel bei einer Temperatur von 25 °C und Normaldruck (760 mmHg) flüssig ist, wobei es sich bei der Verbindung um Benzylalkohol handelt.

2. Zusammensetzung nach dem vorhergehenden Anspruch, **dadurch gekennzeichnet, dass** es sich bei dem Farbstoff um die Verbindung der Formel (3), spezieller in der Natriumsalz-Form, handelt.

3. Zusammensetzung nach einem der vorhergehenden Ansprüche, **dadurch gekennzeichnet, dass** der saure Direktfarbstoff bzw. die sauren Direktfarbstoffe 0,01 bis 20 Gew.-%, vorzugsweise 0,05 bis 10 Gew.-%, noch besser 0,1 bis 5 Gew.-%, des Gesamtgewichts der kosmetischen Zusammensetzung ausmachen.

4. Zusammensetzung nach einem der vorhergehenden Ansprüche, **dadurch gekennzeichnet, dass** die organische Verbindung bzw. die organischen Verbindungen (II) 0,1 bis 20 Gew.-%, vorzugsweise 1 bis 5 Gew,-%, des Gesamtgewichts der Zusammensetzung ausmachen.

5. Zusammensetzung nach einem der vorhergehenden Ansprüche, **dadurch gekennzeichnet, dass** die Zusammensetzung neben den sauren Direktfarbstoffen einen oder mehrere zusätzliche Farbstoffe, die aus natürlichen Farbstoffen oder nicht natürlichen Direktfarbstoffen, Oxidationsfarbstoff-Vorstufen oder Kombinationen davon ausgewählt sind, umfasst.

6. Zusammensetzung nach einem der vorhergehenden Ansprüche, **dadurch gekennzeichnet, dass** sie neben den organischen Verbindungen (II) Wasser und gegebenenfalls ein oder mehrere zusätzliche organische Lösungsmittel umfasst.

7. Zusammensetzung nach einem der vorhergehenden Ansprüche, **dadurch gekennzeichnet, dass** sie ein oder mehrere Oxidationsmittel, vorzugsweise Wasserstoffperoxid, umfasst.

8. Färbeverfahren, das darin besteht, dass man auf Keratinfasern, insbesondere menschliche Keratinfasern, wie das Haar, eine Zusammensetzung gemäß einem der vorhergehenden Ansprüche aufbringt.

9. Vorrichtung mit mehreren Kompartimenten, wobei eines der Kompartimente eine Zusammensetzung nach einem der Ansprüche 1 bis 6 enthält und ein zweites Kompartiment eine oxidierend wirkende Zusammensetzung enthält.

## Revendications

1. Composition tinctoriale pour colorer des fibres kératiniques humaines comprenant :

   - (a) un ou plusieurs colorants directs acides choisis parmi les composés suivants, ainsi que leurs mélanges :

| | |
|---|---|
| 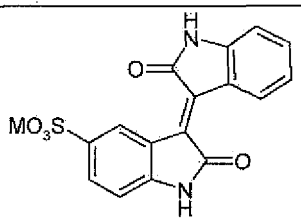 (1) | 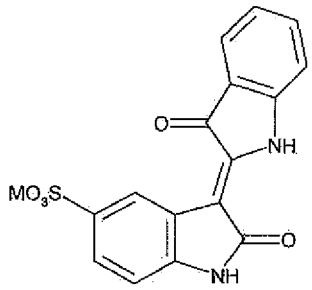 (2) |
| 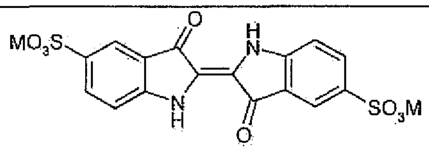 (3) | 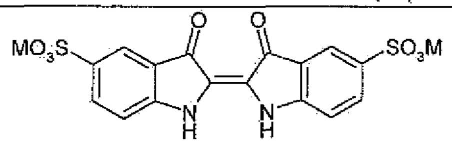 (4) |
| 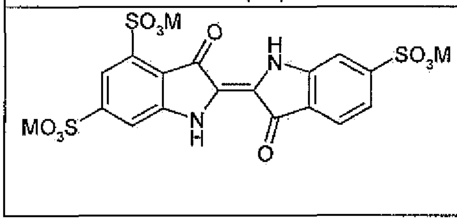 (5) | 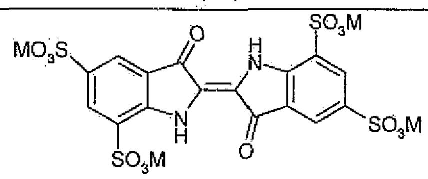 (6) |

M représentant un atome d'hydrogène, un ion sodium ou un ion potassium, la teneur en ce(s) colorant(s) direct(s) acide(s) étant d'au moins 0,01 % en poids par rapport au poids de la composition ; et

- (b) un ou plusieurs composés organiques (II) présentant une valeur du paramètre de solubilité δH de Hansen comprise entre 5 et 16 $MPa^{1/2}$, tel que défini dans le « Handbook of Solubility Parameters and Other Cohesion Parameters », CRC Press, pages 95 à 121 et pages 177 à 185, et dont le poids moléculaire est inférieur à 250 g/mol, à 25 ºC, ledit solvant étant liquide à une température de 25 ºC et à la pression atmosphérique (760 mmHg), ledit composé étant l'alcool benzylique.

2. Composition selon la revendication précédente, **caractérisée en ce que** le colorant est le composé (3) plus particulièrement sous la forme de sel de sodium.

3. Composition selon l'une quelconque des revendications précédentes, **caractérisée en ce que** le ou les colorants directs acides représentent de 0,01 à 20 % en poids, de préférence de 0,05 à 10 % en poids, mieux de 0,1 à 5 % en poids du poids total de la composition cosmétique.

4. Composition selon l'une quelconque des revendications précédentes, **caractérisée en ce que** le ou les composés organiques (II) représentent de 0,1 à 20 % en poids, de préférence de 1 à 5 % en poids, du poids total de la composition.

5. Composition selon l'une quelconque des revendications précédentes, **caractérisée en ce que** la composition comprend un ou plusieurs colorants additionnels différents des colorants directs acides, choisis parmi les colorants naturels et les colorants directs non naturels, les précurseurs de colorant d'oxydation, ou leurs combinaisons.

6. Composition selon l'une quelconque des revendications précédentes, **caractérisée en ce qu'**elle comprend de l'eau et éventuellement un ou plusieurs solvants organiques additionnels différents desdits composés organiques (II).

7. Composition selon l'une quelconque des revendications précédentes, **caractérisée en ce qu'**elle comprend un ou plusieurs agents oxydants, de préférence le peroxyde d'hydrogène.

8. Procédé de coloration consistant à appliquer sur les fibres kératiniques, notamment les fibres kératiniques humaines telles que les cheveux, une composition telle que définie dans l'une quelconque des revendications précédentes.

9. Dispositif à plusieurs compartiments dans lequel l'un des compartiments renferme une composition selon l'une

quelconque des revendications 1 à 6, et un deuxième compartiment renferme une composition oxydante.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9515144 A
- WO 950177 A
- EP 714954 A
- FR 2189006
- FR 2285851
- FR 2140205
- EP 1378544 A
- EP 1674073 A
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136
- EP 0173109 A


### Non-patent literature cited in the description


- **CHARLES M. ; HANSEN.** Hansen solubility parameters: A user's handbook. CRC Press, 2000, 167-185
- Handbook of Solubility Parameters and Other Cohesion Parameters. CRC Press, 95-121, 177-185
- **BAGDA E. ; FARBE LACK.** *The relation between surface tension and solubility parameter in liquids,* 1978, vol. 84, 212
- **WALTER NOLL.** Chemistry and Technology of Silicones. Academic Press, 1968
- **G. FONNUM ; J. BAKKE ; FK. HANSEN.** *Colloid Polym. Sci.,* 1993, vol. 271, 380-389